(19) Europäisches Patentamt
European Patent Office
Office européen des brevets

(11) Numéro de publication : **0 135 510**
**B1**

(12) # FASCICULE DE BREVET EUROPÉEN

(45) Date de publication du fascicule du brevet : **20.08.86**

(51) Int. Cl.⁴ : **A 61 K 31/15**

(21) Numéro de dépôt : **84900517.8**

(22) Date de dépôt : **27.01.84**

(86) Numéro de dépôt international : **PCT/FR 84/00018**

(87) Numéro de publication internationale : **WO/8402844 (02.08.84 Gazette 84/18)**

(54) **NOUVEAUX MEDICAMENTS APPARTENANT A LA FAMILLE DES DERIVES D'HALOGENOBENZOPHENONE-OXIME.**

(30) Priorité : **28.01.83 FR 8301365**

(43) Date de publication de la demande : **03.04.85 Bulletin 85/14**

(45) Mention de la délivrance du brevet : **20.08.86 Bulletin 86/34**

(84) Etats contractants désignés : **AT BE CH DE FR GB LI LU NL SE**

(56) Documents cités : **DE-A- 2 461 039**

(73) Titulaire : **INSTITUT DE RECHERCHES CHIMIQUES ET BIOLOGIQUES APPLIQUEES (I.R.C.E.B.A.) Société à responsabilité limitée dite: 28 rue de Téhéran F-75008 Paris (FR)**

(72) Inventeur : **DANREE, Bernard 59 bis, boulevard Deveaux F-78300 Poissy (FR)**

(74) Mandataire : **Clisci, Serge et al S.A. FEDIT-LORIOT CONSEILS EN PROPRIETE INDUSTRIELLE 38, avenue Hoche F-75008 Paris (FR)**

**EP 0 135 510 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne l'utilisation de dérivés d'halogénobenzophénone-oxime en tant que médicament, notamment en tant qu'agents antibactériens.

On connaît de DE-A-2 518 631 des dérivés d'halogénobenzophénone-oxime en tant que produits industriels utiles pour complexer ou chelater les métaux tels que le cuivre.

On sait, par ailleurs, que l'on a déjà préconisé dans le passé d'utiliser des 2-(halogénobenzyl)-4-alkylphénols (où le groupe 4-alkyle est un radical hydrocarboné ramifié, notamment un radical isopropyle, s-butyle, t-butyle ou 1,1,3,3-tétraméthylbutyle) en tant que substances bactériostatiques, voir à cet effet les brevets américains n° 3 830 852, 3 855 317 et 3 984 482, d'une part, et l'article de BUU-HOI et al., J. Org. Chem., *20*, 1129-1134 (1955), d'autre part.

On sait en particulier que le 2-(2,4-dichlorobenzyl)-4-(1,1,3,3-tétraméthylbutyl)-phénol (qui a pour n° de code B.11 et qui est décrit à l'exemple 1 du brevet américain n° 3 830 852 précité) a été commercialisé en FRANCE en tant que médicament anti-infectieux (Dénomination Commune Internationale : « CLOFOCTOL » ; nom de marque de la spécialité : « OCTOFENE »). Or, il se trouve que ce produit, qui est particulièrement efficace dans le traitement des maladies infectieuses dues aux bactéries $Gram^+$ et ses analogues du type 2-(halogénobenzyl)-4-alkylphénol, ne sont pas naturellement hydrosolubles ni hydrodispersables. La faible affinité pour l'eau constitue un inconvénient qui limite, sur le plan galénique, l'utilisation de ces produits.

Selon l'invention, on préconise en tant que nouveaux médicaments des composés appartenant à la famille des dérivés d'halogénobenzophénone-oxime, qui sont (i) structurellement différents des 2-(halogénobenzyl)-4-alkylphénols précités, d'une part, et particulièrement intéressants sur le plan thérapeutique en raison de leur affinité pour l'eau et de leurs propriétés bactériostatiques et bactéricides, d'autre part, et (ii) nouveaux ou déjà décrits en tant qu'agents complexant les métaux dans DE-A-2 518 631.

Les nouveaux médicaments selon l'invention, qui appartiennent à la famille des dérivés d'halogéno-benzophénone-oxime, sont caractérisés en ce qu'ils répondent à la formule générale :

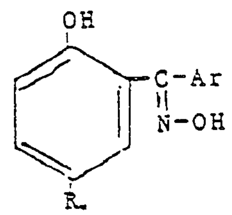

(I)

où R est un groupe alkyle en $C_3$-$C_8$ à chaîne hydrocarbonée ramifiée, et Ar représente un groupe halogénophényle de formule

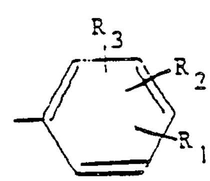

(II)

où $R_1$ est F, Cl ou Br, et $R_2$ et $R_3$, identiques ou différents, représentent chacun H, F, Cl ou Br.

Parmi les groupes alkyle R présentant un reste hydrocarboné ramifié en $C_3$-$C_8$, qui conviennent selon l'invention, on peut notamment citer les groupes $CH(CH_3)_2$, $C(CH_3)_3$, $CH(CH_3)CH_2CH_3$, $CH_2C(CH_3)_3$, $CH_2CH(CH_3)CH_2CH_3$, $C(CH_3)_2CH_2C(CH_3)_3$.

Les produits préférés selon l'invention sont ceux où R est isopropyle, tertiobutyle, néopentyle ou 1,1,3,3-tétraméthylbutyle, et $R_1$ est 4-Cl, $R_2$ est H et $R_3$ est H, 2-Cl ou 3-Cl. Ces produits préférés peuvent être représentés par la formule

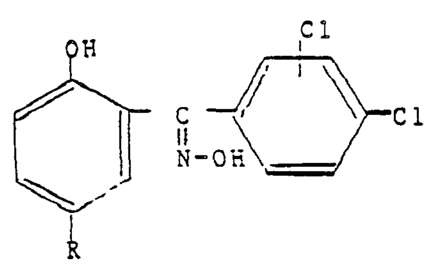

(III)

où R est $CH(CH_3)_2$, $C(CH_3)_3$, $CH_2C(CH_3)_3$ ou $C(CH_3)_2CH_2C(CH_3)_3$. Parmi ceux-ci, le plus intéressant sur

le plan thérapeutique est le 2',4'-dichloro-2-hydroxy-5-(1,1,3,3-tétraméthylbutyl)-benzophénone-oxime.

Un certain nombre de composés utiles comme médicaments selon l'invention a été consigné dans le tableau I ci-après.

Tableau I

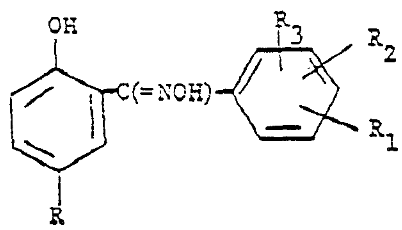

| Produit | R | $R_1$ | $R_2$ | $R_3$ |
|---|---|---|---|---|
| Exemple 1 | $C(CH_3)_2CH_2C(CH_3)_3$ | 4-Cl | H | 2-Cl |
| Exemple 2 | $C(CH_3)_2CH_2C(CH_3)_3$ | 4-Cl | H | 3-Cl |
| Exemple 3 | $CH_2C(CH_3)_3$ | 4-Cl | 3-Cl | 2-Cl |
| Exemple 4 | $CH_2C(CH_3)_3$ | 4-Cl | H | H |
| Exemple 5 | $C(CH_3)_3$ | 4-Cl | H | 2-Cl |
| Exemple 6 | $C(CH_3)_3$ | 4-Cl | H | 3-Cl |
| Exemple 7 | $CH(CH_3)_2$ | 4-Cl | 3-Cl | 2-Cl |
| Exemple 8 | $C(CH_3)_3$ | 4-F | H | H |
| Exemple 9 | $C(CH_3)_3$ | 4-Br | H | 2-Br |
| Exemple 10 | $C(CH_3)_2CH_2C(CH_3)_3$ | 4-F | H | 2-Cl |
| Exemple 11 | $CH_2C(CH_3)_3$ | 4-Cl | H | 2-Cl |
| Exemple 12 | $C(CH_3)_3$ | 4-F | 3-F | 2-F |
| Exemple 13 | $CH_2C(CH_3)_3$ | 4-Br | 3-Br | 2-Br |

Les dérivés d'halogénobenzophénone-oxime de formule I peuvent être préparés selon une méthode connue en soi par application de mécanismes réactionnels classiques.

Le procédé, préconisé selon l'invention pour préparer un composé de formule I, consiste à faire réagir un 2-(halogénobenzoyl)-4-alkyl-phénol de formule

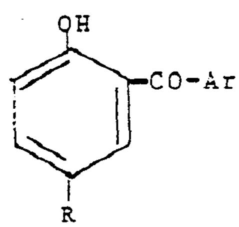

(IV)

(où R et Ar sont définis comme indiqués ci-dessus) avec l'hydroxylamine.

Le meilleur mode de mise en œuvre de ce procédé comprend la réaction d'un composé de formule IV avec le chlorhydrate d'hydroxylamine, dans de la pyridine, pendant au moins 10 heures à la température de reflux du milieu réactionnel, à raison d'au moins 5 moles de chlorhydrate d'hydroxylamine pour 1 mole de 2-(halogénobenzoyl)-4-alkyl-phénol IV.

Les composés de formule IV qui interviennent ici comme matières premières sont obtenus selon le procédé décrit par la Société LABORATOIRES DEBAT dans sa demande de brevet français N° 2 540 103 déposée le 28 janvier 1983.

Selon l'invention, on préconise également une composition thérapeutique qui est caractérisée en ce qu'elle renferme, en association avec un excipient physiologiquement acceptable, au moins un produit de formule I en tant qu'ingrédient actif.

Une telle composition, qui renferme une quantité pharmaceutiquement efficace d'ingrédient actif, peut être administrée par voie orale, rectale ou injectable. Les produits de formule I, qui ont des propriétés bactériostatiques et bactéricides, sont plus précisément indiqués pour le traitement des maladies infectieuses dues aux bactéries Gram⁺, d'une part, et de quelques bactéries Gram⁻ telles que les Neisseria, d'autre part.

D'autres avantages et caractéristiques de l'invention seront mieux compris à la lecture qui va suivre d'exemples de préparation.

Preparation I.

Obtention du 2',4'-dichloro-2-hydroxy-5-(1,1,3,3-tétraméthylbutyl)-benzophénone-oxime.

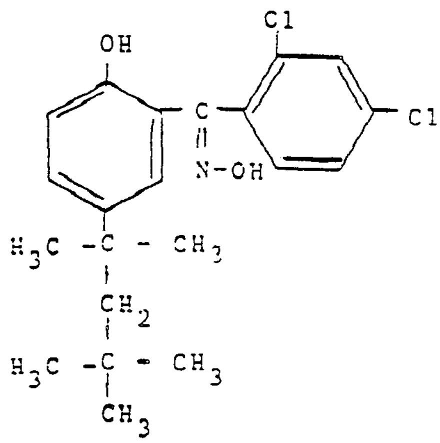

(exemple 1 ; N° de code B-602).

Dans un tricol de 500 ml muni d'un réfrigérant ascendant, d'un thermomètre, d'un dispositif d'agitation et d'ampoules d'introduction, on charge sous agitation 37,93 g (0,1 mole) de 2-(2,4-dichlorobenzoyl)-4-(1,1,3,3-tétraméthylbutyl)-phénol et 180 ml de pyridine. On ajoute ensuite 52,54 g (0,756 mole) de chlorhydrate d'hydroxylamine tout en maintenant l'agitation (la réaction est initialement exothermique). On porte ensuite le milieu réactionnel au reflux pendant 20 heures. On élimine la pyridine par évaporation sous vide à 90 °C, et reprend le résidu d'évaporation ainsi obtenu par 200 ml d'eau. On extrait la phase organique par 3 × 150 ml d'éther diéthylique. Les phases éthérées rassemblées sont lavées par 2 × 250 ml HCl IN puis par $H_2O$ jusqu'à neutralité, et enfin séchées sur $Na_2SO_4$. Par concentration sous vide, on obtient 38,6 g (rendement = 98 %) d'un liquide jaunâtre cristallisant à froid et présentant en chromatographie sur couche mince [plaque de silice ; éluant : toluène-acétate d'éthyle (95 : 5)v/v] deux tâches.

Par recristallisation dans le cyclohexane, on isole 31,9 g (rendement : 81 %) de 2',4'-dichloro-2-hydroxy-5-(1,1,3,3-tétraméthylbutyl)-benzophénone-oxime, qui est monotache en CCM. $F_{inst.}$ = 146 °C.

Preparation II.

Obtention du 2'4'-dichloro-2-hydroxy-5-tertiobutyl-benzophénone-oxime (exemple 5).

En procédant comme indiqué dans la préparation I mais en remplaçant le 2-(2,4-dichlorobenzoyl)-4-(1,1,3,3-tétraméthylbutyl)-phénol par le 2-(2,4-dichlorobenzoyl)-4-tertiobutyl-phénol, on obtient le 2'4'-dichloro-2-hydroxy-5-tertiobutyl-benzophénone-oxime.

On a résumé ci-après les résultats qui ont été entrepris avec le produit de l'exemple 1 (B.602) préféré selon l'invention, et le CLOFOCTOL (B.11), en tant que produit de référence.

A. Toxicité et tolérance.

Les essais ont été effectués sur des lots de souris (mâles et femelles) pesant chacune 18 à 20 g et recevant les produits à tester aux doses de 0,5 g/kg, 0,8 g/kg, 1 g/kg, 2 g/kg, 3 g/kg et 4 g/kg per os dans un milieu aqueux renfermant 20 g/l de carboxyméthylcellulose, les animaux étant observés pendant les 14 jours suivant l'administration.

On a observé que :

1. chez la souris, per os, le B.602 (DL-50 > 4 g/kg) est moins toxique que le B.11 (DL-50 = 2 g/kg) ;

2. le B.602 est mieux toléré que le B.11, notamment en ce qui concerne certains effets secondaires :

pas de diarrhée à la dose de 4 g/kg pour le B.602, alors que le B.11 induit une diarrhée dès la dose de 0,8 g/kg ;

pas de modification de la température rectale par le B.602 aux doses de 2 g/kg et 4 g/kg alors que le B.11 diminue pendant 48 heures la température rectale dès la dose de 2 g/kg.

pas de modification pondérale à la dose de 2 g/kg pour le B.602 alors que le B.11 induit une légère hypertrophie hépatique à partir de 0,8 g/kg.

B. Activité antibactérienne.

L'activité antibactérienne a été étudiée in vitro, selon la technique usuelle des dilutions, vis à vis de plusieurs souches, de façon à déterminer la concentration minimale inhibitrice (en abrégé M.I.C.) c'est-à-dire la concentration minimale bactériostatique.

Les résultats de cette étude sont consignés dans le tableau II ci-après, les souches utilisées étant celles des catalogues de « l'Institut Pasteur » (en abrégé CIP), du « Centre International de Distribution de Souches et d'Information sur les Types Microbiens de Lausanne » (en abrégé La) et de l'« American Type Culture Collection » (en abrégé ATCC).

Il résulte des essais qui ont été entrepris que le B.602 selon l'invention est particulièrement intéressant vis à vis des souches Gram$^+$ et qu'il est plus actif que le B.11. Par ailleurs, le B.602 est également actif vis à vis de quelques souches Gram$^-$ telles que les *Neisseria.*

Tableau II

Activité antibactérienne (MIC en µg/ml)

| SOUCHES BACTERIENNES | Exemple 1 (B.602) MIC | Clofoctol (B.11) MIC |
|---|---|---|
| Staphylococcus aureus London CIP A.238 | 1 | 3 |
| Staphylococcus aureus La 634 | 3 | 6 |
| Staphylococcus aureus La 636 | 4 | 5 |
| Staphylococcus aureus La 659 | 5 | 6 |
| Staphylococcus aureus ATCC 6538 P | 4 | 8 |
| Streptococcus pyogenes CIP. A 241 | 2 | 4 |
| Streptococcus pyogenes CIP. 56.1 | 2 | 3 |
| Streptococcus pyogenes CIP. 56.41 | 3 | 4 |
| Streptococcus pyogenes CIP. 56.43 | 1 | 2 |
| Streptococcus pyogenes CIP. 56.45 | 1 | 2 |
| Streptococcus La 147 | 2 | 7 |
| Streptococcus faecalis CIP 53.152 | 3 | 6 |
| Streptococcus faecalis CIP 55.142 | 2 | 5 |
| Diplococcus pneumoniae La 209 | 4 | 4 |
| Diplococcus pneumoniae La 210 | 3 | 5 |
| Bacillus subtilis ATCC 6633 | 1 | 4 |

**Revendications** (pour les Etats contractants : BE, CH, DE, FR, GB, LI, LU, NL, SE)

1. Médicament appartenant à la famille des dérivés d'halogénobenzophénone-oxime, caractérisé en ce qu'il répond à la formule générale

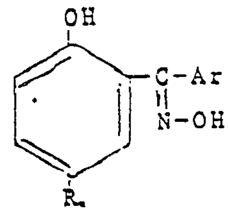

(I)

où R est un groupe alkyle en $C_3$-$C_8$ à chaîne hydrocarbonée ramifiée, et Ar représente un groupe halogénophényle de formule

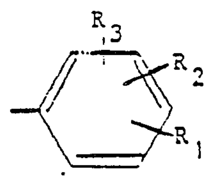

(II)

où $R_1$ est F, Cl ou Br, et $R_2$ et $R_3$, identiques ou différents, représentent chacun H, F, Cl ou Br.

2. Médicament selon la revendication 1, caractérisé en ce qu'il est choisi parmi l'ensemble constitué par les composés de formule

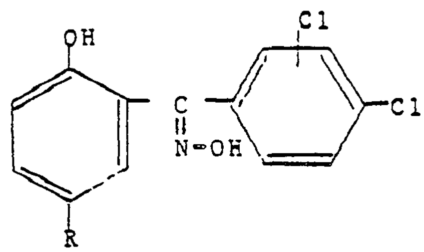

(III)

où R est $CH(CH_3)_2$, $C(CH_3)_3$, $CH_2C(CH_3)_3$ ou $C(CH_3)_2CH_2C(CH_3)_3$.

3. Médicament selon la revendication 2, caractérisé en ce qu'il s'agit du 2'4'-dichloro-2-hydroxy-5-(1,1,3,3-tétraméthylbutyl)-benzophénone-oxime.

4. Composition thérapeutique caractérisée en ce qu'elle renferme, en association avec un excipient physiologiquement acceptable, au moins un dérivé d'halogénobenzophénone-oxime, selon l'une quelconque des revendications 1 à 3.

5. Procédé de préparation d'un dérivé d'halogénobenzophénone-oxime utile comme médicament et répondant à la formule générale

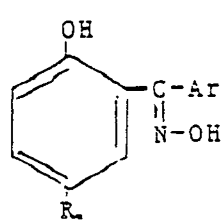

(I)

dans laquelle R est un groupe alkyle en $C_3$-$C_8$ à chaîne hydrocarbonée ramifiée, et Ar représente un groupe halogénophényle de formule

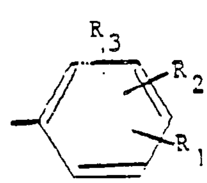

(II)

où $R_1$ est F, Cl ou Br, et $R_2$ et $R_3$, identiques ou différents, représentent chacun H, F, Cl ou Br ; caractérisé en ce que l'on fait réagir une mole de 2-(halogénobenzoyl)-4-alkylphénol de formule

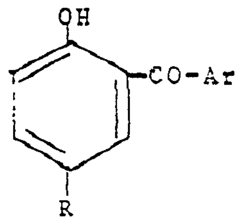

(IV)

où R et Ar sont définis comme ci-dessus, avec au moins 5 moles de chlorhydrate d'hydroxylamine, dans de la pyridine pendant au moins 10 heures à la température de reflux du milieu réactionnel.

**Revendications** (pour l'Etat contractant AT)

1. Procédé de préparation d'un dérivé d'halogénobenzophénone-oxime utile comme médicament et répondant à la formule générale

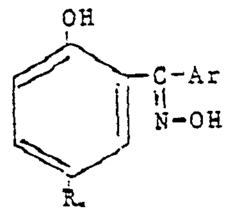

(I)

dans laquelle R est un groupe alkyle en $C_3$-$C_8$ à chaîne hydrocarbonée ramifiée, et Ar représente un groupe halogénophényle de formule

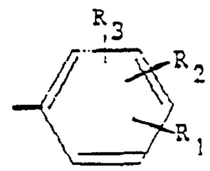

(II)

où $R_1$ est F, Cl ou Br, et $R_2$ et $R_3$, identiques ou différents, représentent chacun H, F, Cl ou Br ; caractérisé en ce que l'on fait réagir une mole de 2-(halogénobenzoyl)-4-alkylphénol de formule

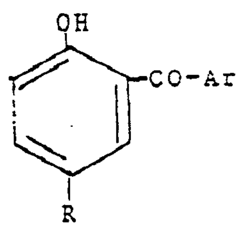

(IV)

où R et Ar sont définis comme ci-dessus, avec au moins 5 moles de chlorhydrate d'hydroxylamine, dans de la pyridine pendant au moins 10 heures à la température de reflux du milieu réactionnel.

2. Procédé selon la revendication 1, caractérisé en ce que R est $CH(CH_3)_2$, $C(CH_3)_3$, $CH_2C(CH_3)_3$ ou $C(CH_3)_2CH_2C(CH_3)_3$ et Ar est

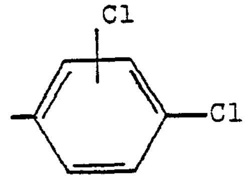

3. Procédé selon la revendication 1, caractérisé en ce que R est $C(CH_3)_2CH_2C(CH_3)_3$ et Ar est 2,4-dichlorophényle.

**Claims** (for the Contracting States : BE, CH, DE, FR, GB, LI, LU, NL, SE)

1. A drug belonging to the halogenobenzophenone-oxime derivatives family, characterised in that it is selected from the group consisting of compounds of the general formula

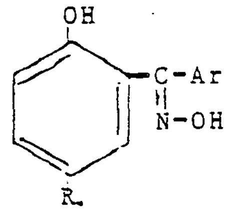

(I)

wherein R is a $C_3$-$C_8$ alkyl group with a branched hydrocarbon chain and Ar represents a halogenophenyl group of the formula

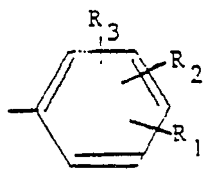

(II)

wherein $R_1$ is F, Cl or Br, and $R_1$ and $R_2$, which may be identical or different, represent each H, F, Cl or Br.

2. A drug according to claim 1, characterised in that it is selected from the group consisting of compounds of the formula

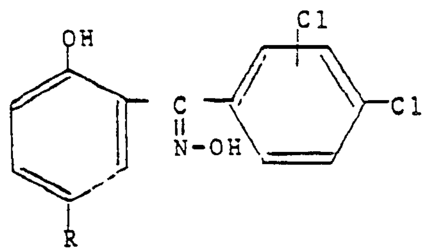

(III)

wherein R is $CH(CH_3)_2$, $C(CH_3)_3$, $CH_2C(CH_3)_3$ or $C(CH_3)_2CH_2C(CH_3)_3$.

3. A drug according to claim 1 which 2',4'-dichloro-2-hydroxy-5-(1,1,3,3-tetramethylbutyl)-benzophenone-oxime.

4. A therapeutical composition characterised in that it comprises, in association with a physiologically acceptable excipient, a pharmaceutically effective amount of a halogenobenzophenone-oxime derivative of formula I according to claim 1.

5. A method for preparing a halogenobenzophenone-oxime derivative useful in therapeutics as antibacterial agent and having the general formula

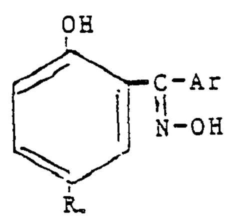

(I)

wherein R is a $C_3$-$C_8$ alkyl group with a branched hydrocarbon chain and Ar represents a halogenophenyl group of the formula

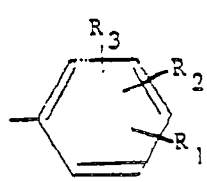

(II)

in which $R_1$ is F, Cl or Br, and $R_1$ and $R_2$, which may be identical or different, represent each H, F, Cl or Br, said method comprising reacting 1 mol of 2-(halogenobenzoyl)-4-alkylphenol of the formula

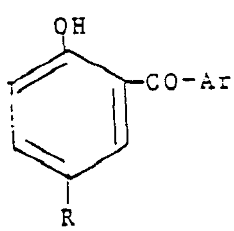

(IV)

(wherein R and Ar are as above defined) with at least 5 mols of hydroxylamine hydrochloride in pyridine for at least 10 hours at the reflux temperature of the reaction medium.

**Claims** (for the Contracting State AT)

1. A method for preparing a halogenobenzophenone-oxime derivative useful in therapeutics as antibacterial agent and having the general formula

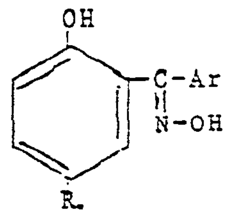

(I)

wherein R is a $C_3$-$C_8$ alkyl group with a branched hydrocarbon chain and Ar represents a halogenophenyl group of the formula

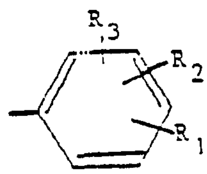

(II)

in which $R_1$ is F, Cl or Br, and $R_1$ and $R_2$, which may be identical or different, represent each H, F, Cl or Br, said method comprising reacting 1 mol of 2-(halogenobenzoyl)-4-alkylphenol of the formula

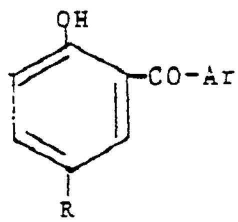

(IV)

(wherein R and Ar are as above defined) with at least 5 mols of hydroxylamine hydrochloride in pyridine for at least 10 hours at the reflux temperature of the reaction medium.

2. A method according to claim 1, wherein R is $CH(CH_3)_2$, $C(CH_3)_3$, $CH_2C(CH_3)_3$ or $C(CH_3)_2CH_2C(CH_3)_3$, and Ar is

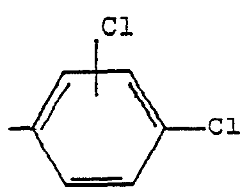

3. A method according to claim 1, wherein R is $C(CH_3)_2CH_2C(CH_3)_3$ and Ar is 2,4-dichlorophenyl.

**Patentansprüche** (für die Vertragsstaaten BE, CH, DE, FR, GB, LI, LU, NL, SE)

1. Medikament, gehörend zu der Familie der Derivate des Halogenbenzophenonoxims, dadurch gekennzeichnet, daß es der allgemeinen Formel

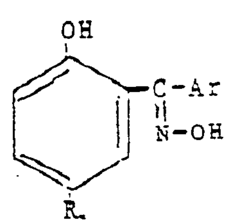

(I)

entspricht, in der R eine Alkylgruppe mit einer verzweigten $C_3$-$C_8$-Kohlenwasserstoffkette bedeutet und Ar eine Halogenphenylgruppe der Formel

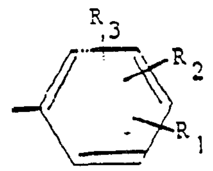

(II)

darstellt, worin $R_1$ gleich F, Cl oder Br ist und $R_2$ und $R_3$ gleich oder verschieden sind und jeweils H, F, Cl oder Br darstellen.

2. Medikament nach Anspruch 1, dadurch gekennzeichnet, daß es ausgewählt ist aus der Gruppe, bestehend aus den Verbindungen der Formel

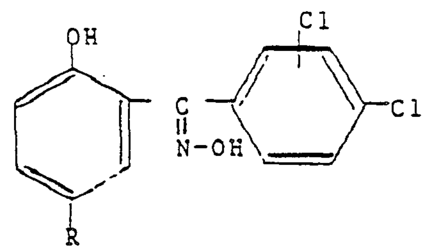

(III)

worin R gleich $CH(CH_3)_2$, $C(CH_3)_3$, $CH_2C(CH_3)_3$ oder $C(CH_3)_2CH_2C(CH_2)_3$ ist.

3. Medikament nach Anspruch 2, dadurch gekennzeichnet, daß es sich um 2',4'-Dichlor-2-hydroxy-5-(1,1,3,3-tetramethylbutyl)-benzophenonoxim handelt.

4. Therapeutische Zusammensetzung, dadurch gekennzeichnet, daß sie in Verbindung mit einem pharmazeutisch akzeptierbaren Träger mindestens ein Derivat des Halogenbenzophenonoxims nach einem der Ansprüche 1 bis 3 enthält.

5. Verfahren zur Herstellung eines Derivats des Halogenbenzophenonoxims, das als Medikament verwendet werden kann und der allgemeinen Formel

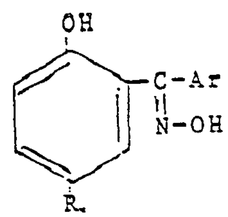

(I)

entspricht, in welcher R gleich einer Alkylgruppe mit einer verzweigten $C_3$-$C_8$-Kohlenwasserstoffkette ist und Ar eine Halogenphenylgruppe der Formel

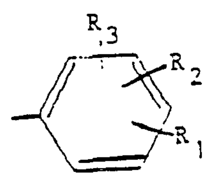

(II)

darstellt,
worin R gleich F, Cl oder Br ist und $R_2$ und $R_3$ gleich oder verschieden sind und jeweils H, F, Cl oder Br darstellen ; dadurch gekennzeichnet, daß man ein Mol 2-(Halogenbenzoyl)-4-alkylphenol der Formel

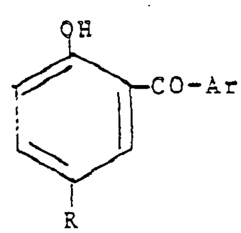

(IV)

worin R und Ar wie oben definiert sind, mit mindestens 5 Molen Hydroxylaminchlorhydrat mindestens 10 Stunden lang bei der Rückflußtemperatur der Reaktionsmischung in Pyridin umsetzt.

**Patentansprüche** (für den Vertragsstaat AT)

1. Verfahren zur Herstellung eines Derivats des Halogenbenzophenonoxims, das als Medikament verwendbar ist und der allgemeinen Formel

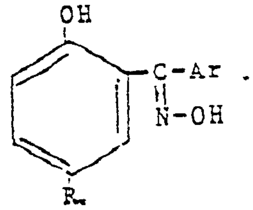

(I)

entspricht, in welcher R eine Alkylgruppe mit einer verzweigten $C_3$-$C_8$-Kohlenwasserstoffkette ist und R eine Halogenphenylgruppe der Formel

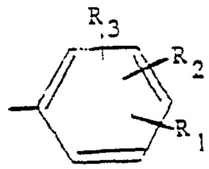

(II)

darstellt, worin $R_1$ gleich F, Cl oder Br ist und $R_2$ und $R_3$ gleich oder verschieden sind und jeweils H, F, Cl oder Br darstellen, dadurch gekennzeichnet, daß man ein Mol 2-(Halogenbenzoyl)-4-alkylphenol der Formel

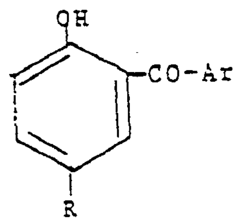

(IV)

worin R und Ar wie oben definiert sind, mit mindestens 5 Molen Hydroxylaminchlorhydrat mindestens 10 Stunden lang bei der Rückflußtemperatur der Reaktionsmischung in Pyridin umsetzt.

2. Verfahren nach Anspruch 1, dadurch gekennzeichnet, daß R gleich $CH(CH_3)_2$, $C(CH_3)_3$, $CH_2C(CH_3)$ oder $C(CH_3)_2CH_2C(CH_3)$ und Ar gleich

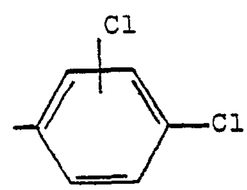

ist

3. Verfahren nach Anspruch 1, dadurch gekennzeichnet, daß R gleich $C(CH_3)_2CH_2C(CH_3)_3$ und Ar gleich 2,4-Dichlorphenyl ist.